# DEMANDE DE BREVET EUROPEEN

(11) **EP 1 618 865 A1**
(43) Date de publication de la demande: **25.01.2006**
(21) Numéro de dépôt: 05291334.0
(22) Date de dépôt: 22.06.2005
(51) Int. Cl.: A61K 8/18

(54) **Utilisation de composés polycationiques en teinture des fibres kératiniques**

(30) Priorité: 23.06.2004 FR 0406848
(71) Demandeur: L'OREAL, 75008 Paris (FR)
(72) Inventeur: Lagrange, Alain, 77700 Coupvray (FR)
(74) Mandataire: Dossmann, Gérard

(57) **Abrégé**

La présente demande a pour objet l'utilisation à titre de colorant direct dans des compositions tinctoriales pour fibres kératiniques, en particulier les fibres kératiniques humaines telles que les cheveux, ou pour la fabrication de telles compositions, d'un composé polycationique particulier.

## Description

La présente demande a pour objet l'utilisation de composés polycationiques particuliers, à titre de colorants directs dans des compositions tinctoriales pour la teinture des fibres kératiniques, et en particulier les cheveux humains. Elle vise également des compositions tinctoriales à base de ces colorants, ainsi que l'utilisation de ces compositions pour la teinture des fibres kératiniques.

Il est connu de teindre les fibres kératiniques et en particulier les cheveux humains avec des compositions tinctoriales contenant des précurseurs de colorant d'oxydation, appelés généralement bases d'oxydation, tels que des ortho- ou para-phénylènediamines, des ortho- ou para-aminophénols et des composés hétérocycliques. Ces bases d'oxydation sont des composés incolores ou faiblement colorés qui, associés à des produits oxydants, peuvent donner naissance par un processus de condensation oxydative à des composés colorés. Ces colorants, insolubles dans le milieu de teinture, sont piégés à l'intérieur du cheveu.

On sait également que l'on peut faire varier les nuances obtenues avec ces bases d'oxydation en les associant à des coupleurs ou modificateurs de coloration, ces derniers étant choisis notamment parmi les méta-diamines aromatiques, les méta-aminophénols, les métadiphénols et certains composés hétérocycliques tels que des composés indoliques.

La variété des molécules mises en jeu au niveau des bases d'oxydation et des coupleurs permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs tels que la lumière, les intempéries, le lavage, les ondulations permanentes, la transpiration et les frottements.

Les colorants doivent également permettre de couvrir les cheveux blancs, et être enfin les moins sélectifs possibles, c'est-à-dire permettre d'obtenir des écarts de coloration les plus faibles possibles tout au long d'une même fibre kératinique, qui est en général différemment sensibilisée (i.e. abîmée) entre sa pointe et sa racine.

Il est aussi connu de teindre les fibres kératiniques par une coloration directe ou semi-permanente. Le procédé classiquement utilisé en coloration directe consiste à appliquer sur les fibres kératiniques des colorants directs qui sont des molécules colorées et colorantes ayant une affinité pour les fibres, à laisser poser pour permettre aux molécules colorées de pénétrer, par diffusion, à l'intérieur du cheveu, puis à rincer les fibres.

Contrairement aux compositions de teinture par oxydation, les compositions de teinture directes ou semi-permanentes peuvent être mises en oeuvre sans la présence d'un agent oxydant. Ces teintures peuvent être effectuées de manière répétée sans dégrader la fibre kératinique.

Il est connu par exemple d'utiliser des colorants directs nitrés benzéniques, anthraquinoniques, nitropyridiniques, azoïques, xanthéniques, acridiniques, aziniques ou triarylméthaniques.

Il en résulte des colorations particulièrement chromatiques qui sont cependant temporaires ou semi-permanentes à cause de la nature des liaisons entre les colorants directs et la fibre kératinique. Ces interactions font que la désorption des colorants de la surface et/ou du coeur de la fibre se fait facilement. Les colorations présentent généralement une faible puissance tinctoriale et une mauvaise tenue aux lavages ou à la transpiration. Ces colorants directs sont en outre généralement sensibles à la lumière car la résistance du chromophore vis-à-vis des attaques photochimiques est faible, ce qui conduit à un affadissement de la coloration des cheveux dans le temps. La sensibilité de ces colorants à la lumière dépend de leur répartition uniforme ou en agrégats dans et/ou sur la fibre kératinique.

Par ailleurs, les colorants directs classiques ne sont pas toujours complètement inoffensifs, c'est pourquoi en cosmétique capillaire, on recherche des molécules colorantes de ce type toujours plus performantes en terme d'innocuité.

Il existe un réel besoin de disposer de compositions de teinture directe améliorées en terme de tenue aux shampooings et de montée du colorant.

De manière surprenante et avantageuse, la Demanderesse vient de découvrir que l'utilisation de composés polycationiques particuliers dans des compositions pour la teinture des fibres kératiniques, en particulier des fibres kératiniques humaines telles que les cheveux, permettait d'obtenir des compositions tinctoriales qui présentent ces améliorations.

Outre leur avantage en terme d'innocuité, les compositions selon la présente demande permettent l'obtention de teintures résistantes aux agents extérieurs (tels que le soleil, les intempéries), ainsi qu'aux shampooings et à la transpiration, et permettent d'obtenir des nuances intenses et tenaces sur les fibres.

La présente invention a ainsi pour objet l'utilisation à titre de colorant direct dans des compositions tinctoriales pour fibres kératiniques, en particulier les fibres kératiniques humaines telles que les cheveux, ou pour la fabrication de telles compositions, de composés de formule (I) décrits ci-dessous.

L'invention a également pour objet une composition tinctoriale pour la teinture des fibres kératiniques, en particulier des fibres kératiniques humaines telles que les cheveux comprenant dans un milieu de teinture approprié au moins une base d'oxydation et au moins un composé de formule (I).

Un autre objet de l'invention porte sur un procédé de teinture des fibres kératiniques, en particulier des fibres kératiniques humaines telles que les cheveux mettant en oeuvre cette composition.

L'invention a également pour objet l'utilisation de la composition de la présente invention sur les fibres kératiniques, en particulier les fibres kératiniques humaines telles que les cheveux pour obtenir des teintures présentant une bonne résistance aux agents extérieurs et aux shampooings. Les formes tautomères des composés de formule (I) sont également utilisables.

D'autres caractéristiques, aspects, objets et avantages de l'invention apparaîtront encore plus clairement à la lecture de la description et des exemples qui suivent.

Les composés utilisables à titre de colorants directs selon l'invention répondent à la formule (I) : 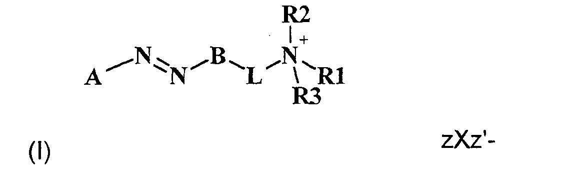
dans laquelle :
X^{z'-} désigne un anion organique ou minéral ;
z et z' sont tels que la charge globale de la molécule soit nulle ;
A désigne un cycle hétérocyclique porteur d'une charge positive ayant la signification thiazolium substitué ou non, les substituants du thiazolium contenant au total au plus une fonction alcoxycarbonyle ; imidazolium substitué ou non ; benzimidazolium substitué ou non ; benzothiazolium pyridino substitué ou non ; naphthothiazolium substitué ou non et benzothiazolium substitué ;
B désigne un groupement arylène en C6-C30 éventuellement substitué ou un groupe hétérocyclique de 5 à 10 chaînons éventuellement substitué ;
L désigne une chaîne alkylène linéaire, ramifiée ou cyclique, substituée ou non et comportant de 1 à 30 atomes de carbone, la chaîne pouvant être ou non interrompue ou terminée par un ou plusieurs groupements hétéroatomiques choisi parmi O, S, NH, NR avec R représentant un radical alkyle en C1-C10 linéaire ou ramifié, ou interrompue par un ou plusieurs radicaux cycliques, aromatiques ou non, hétérocycliques ou non, la chaîne elle-même pouvant être porteuse d'une ou plusieurs charges cationiques quaternaires et un ou plusieurs atomes de carbone de la chaîne pouvant être remplacés par un groupement carbonyle ; de préférence, L contient au moins un groupement -CH₂- ;
R1, R2, R3, désignent indépendamment l'un de l'autre un radical alkyle en C1-C10 linaire, ramifié ou cyclique éventuellement substitué ; un radical arylalkyle en C1-C10 linaire, ramifié éventuellement substitué ; un radical aryle, éventuellement substitué ; un radical aryloxyalkyle en C1-C10 linaire, ramifié ; lesdits groupements aryle étant de préférence en C6 ; l'un au moins des radicaux R1, R2, R3 pouvant former avec l'atome d'azote le(s) portant et éventuellement avec L un hétérocycle saturé ou insaturé ayant de 5 à 10 chaînons éventuellement substitué pouvant contenir un ou plusieurs hétéroatomes supplémentaires.

Selon une variante, deux des radicaux R1, R2 et R3 forment un cycle avec l'atome d'azote les portant et le radical restant forme éventuellement un cycle avec L.

Selon une autre variante, l'un des radicaux R1, R2 ou R3 forme avec l'atome d'azote le portant et avec L un hétérocycle, les deux radicaux restants n'étant pas engagés dans un cycle avec l'atome d'azote les portant.

De préférence, R1, R2 et/ou R3 ne forment pas de cycle avec L.

Par substituant, on entend au sens de la présente invention un radical choisi parmi les radicaux hydroxy, amino, halogène, alkyle en C1-C10, aryle en C6-C30, hydroxyalkyle en C1-C10, mono ou di alkyle(C1-C10)amino, mono ou di hydroxyalkyle(C1-C10)amino, alcoxy(C1-10), amido, phénylamido, carboxy, alcoxy(C1-C10)carbonyle, sulfonato, alcoxy(C1-C10)carbonylealkyle(C1-C10), hétérocycle de 5 à 10 chaînons, hétérocycle alkyle en C1-C10 avec un hétérocycle ayant de 5 à 10 chaînons.

A titre de groupement arylène on peut citer les groupement benzène, naphtalène, anthracène et pérylène.

A titre d'anions organiques ou minéraux on peut citer les ions halogénures et en particulier les ions chlorure, bromure, iodure, les ions méthanesulfonate, les ions méthosulfate, les ions bromate ou chlorate,les ions acétate, les ions sulfate, les ions tartrate, les ions lactate, les ions citrates,les ions chlorozincate.

A titre d'hétérocycle convenant pour le groupement B on peut citer de manière non limitative les cycles thiazole, pyridine et pyrimidine.

De préférence B est un groupement arylène.

Parmi les composés de formule (I), on peut citer les composés suivants :
2-[[4-[éthyl[2-(triméthylammonio)éthyl]amino]phényl]azo]-4-(méthoxycarbonyl)-5-(2-méthoxy-2-oxoéthyl)-3-méthyl-thiazolium 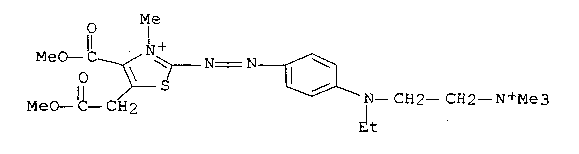
dichlorure de thiazolium, 2-[[4-[[2-[diméthyl(phénylméthyl) ammonio]éthyl]éthylamino]phényl]azo]-3-méthyl-thiazolium 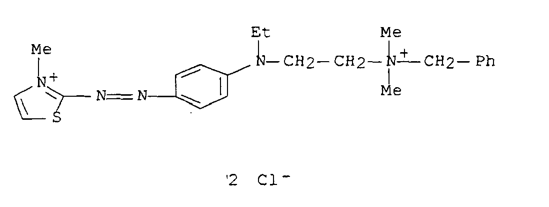
2-[[4-(diéthylamino)-2-[[(triméthylammonio)acétyl]amino] phényl]azo]-5-(éthoxycarbonyl)-3-méthyl-4-phényl-thiazolium 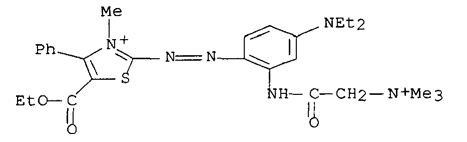
bis[tétrafluoroborate(1-)] de 2-[[4-(diéthylamino)-2-[[(triméthylammonio)acétyl]oxy]phényl]azo]-5-(éthoxycarbonyl)-3,4-diméthyl-thiazolium 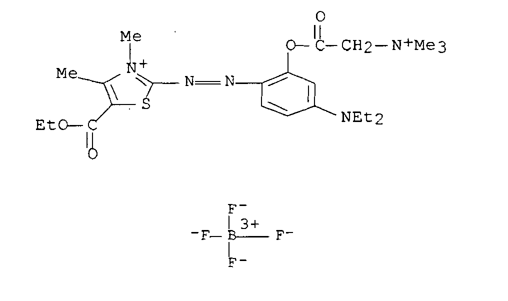
2-[[4-(diéthylamino)-2-[[(triméthylammonio)acétyl]oxy] phényl]azo]-5-(éthoxycarbonyl)-3,4-diméthyl-thiazolium 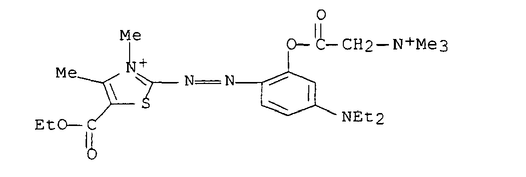
dichlorure de 2-[[4-[éthyl[2-[(2-hydroxypropyl)diméthylammonio]éthyl]amino]-2-méthylphényl]azo]-3-méthyl-thiazolium 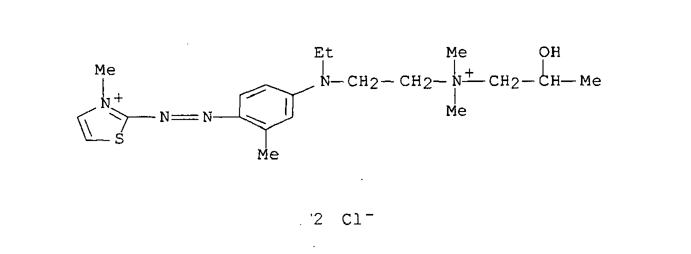
dichlorure de 2-[[4-[éthyl[2-(triméthylammonio)éthyl]amino] phényl]azo]-3-méthyl-thiazolium 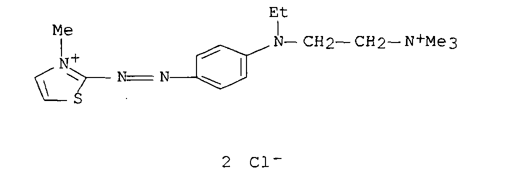
bis[tétrafluoroborate(1-)] de 2-[[4-[éthyl[2-(triméthylammonio) éthyl]amino]phényl]azo]-3-méthyl-4-phényl- thiazolium, 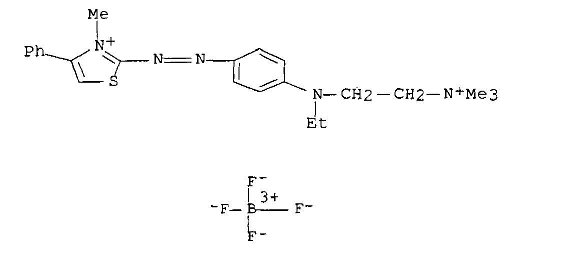
2-[[4-[éthyl[2-(triméthylammonio)éthyl]amino]phényl]azo]-3-méthyl-4-phényl-thiazolium 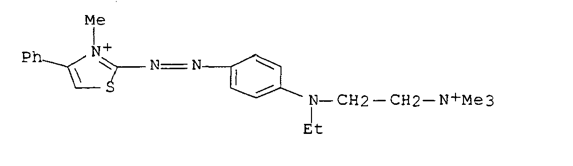
chlorure de 1-(2-{(4-méthoxy-phényl)-[3-méthyl-4-(thiazol-3-ium-2-ylazo)-phényl]-amino}-ethyl)-4-méthyl-pyridinium 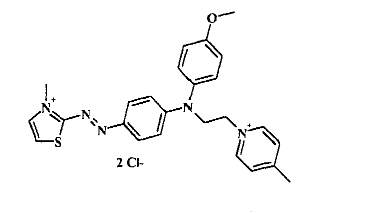
chlorozincate de 1-(2-{phényl-[5-éthoxycarbonyl-3,4-diméthyl-4-(thiazol-3-ium-2-ylazo)-phényl]-amino}-éthyl)-pyridinium 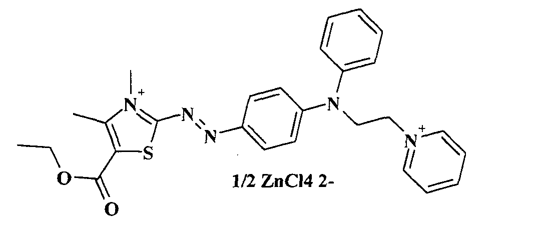
chlorure de 1-(2-{(4-méthoxy-phényl)-[5-éthoxycarbonyl-3,4-diméthyl-4-(thiazol-3-ium-2-ylazo)-phényl]-amino}-éthyl)-4-méthyl-pyridinium 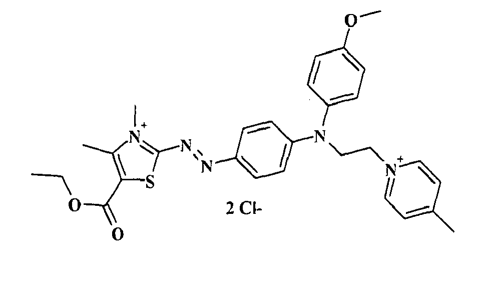
chlorozincate de 4-diméthylamino-1-(2-{[4-(5-éthoxycarbonyl-3,4-diméthyl-4-(thiazol-3-ium-2-ylazo)-phényl]-p-tolyl-amino}-éthyl) pyridinium 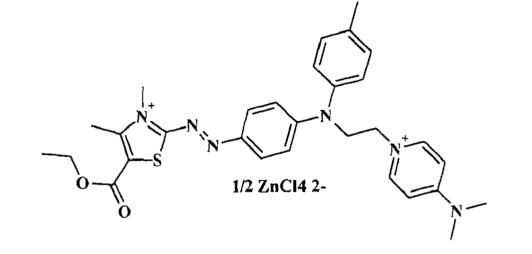
chlorure de 1-{2-[[4-(5-acétyl-3,4-diméthyl-4-(thiazol-3-ium-2-ylazo )-phényl]-(4-méthoxy-phényl)-amino]-éthyl-pyridinium 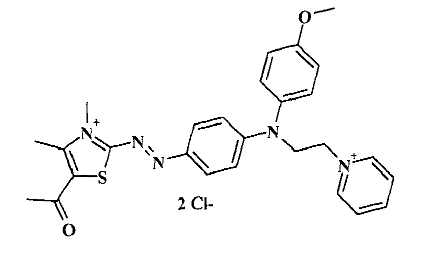
chlorozincate de 1-(3-{[4-(5-éthoxycarbonyl-3,4-diméthyl-4-(thiazol-3-ium-2-ylazo)-phényl]-phényl-amino}-2-hypropyl)-pyridinium 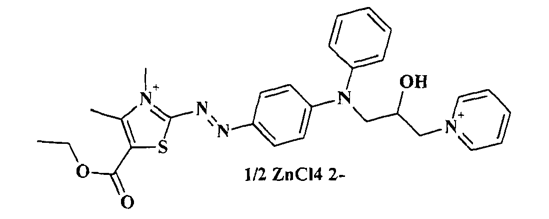
chlorure 1-{3-[[4-(5-éthoxycarbonyl-3,4-diméthyl-4-(thiazol-3-ium-2-ylazo)-phényl]-(4-méthoxy-phényl)-2-hydroxypropyl)-4-méthyl-pyridinium 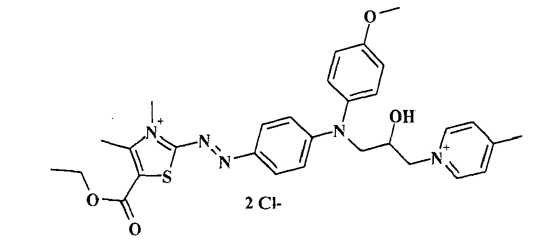
diiodure de 4-[4-[(1,3-diméthyl-1H-imidazolium-2-yl)azo] phényl]-1,1-diméthyl-pipérazinium 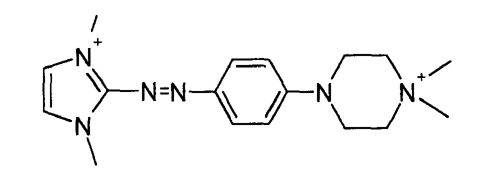
diiodure de 2-[[4-[éthyl[2-[[(triméthylammonio)acétyl]amino] éthyl]amino]-2-méthylphényl]azo]-1,3-diméthyl-1H-imidazolium 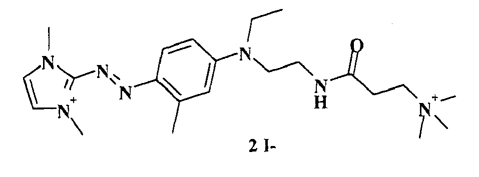
diiodure de 2-[[4-[[3-(diéthylméthylammonio)-2-hydroxypropyl]éthylamino]-2-méthylphényl]azo]-1,3-diméthyl-1H-imidazolium, 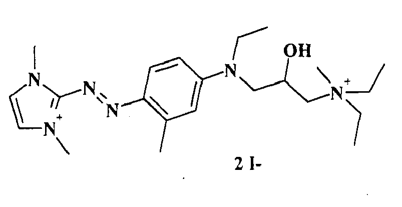
diiodure de 2-[[4-[[2-(diéthylméthylammonio)éthyl] éthylamino]-2-méthylphenyl]azo]-1,3-diméthyl-1H-imidazolium 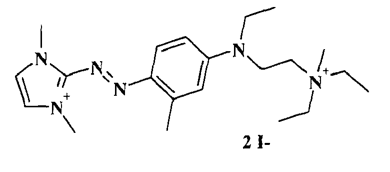
diiodure de 2-[[4-[éthyl[2-(triméthylammonio)éthyl]amino] phényl]azo]-1,3-diméthyl-1H-imidazolium 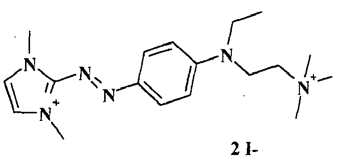
diiodure de 2-[[4-[[2-(diéthylméthylammonio)éthyl] éthylamino]phényl]azo]-1,3-diméthyl-1H-imidazolium 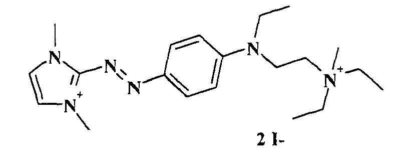
dichlorure de 2-[[4-[éthyl[2-(triméthylammonio)éthyl] amino] phényl]azo]-3-méthyl-naphtho[2,1-d]thiazolium 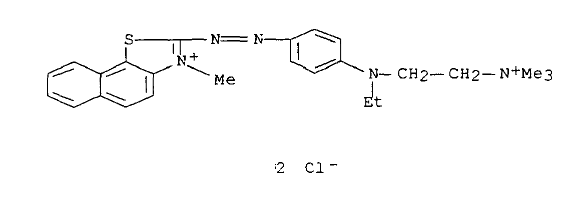
2-[[4-anilino-2-(éthylamino)-6-[[3- (triméthylammonio)propyl] amino]-5-pyrimidinyl]azo]-6-méthoxy-3-méthyl-benzothiazolium, bis(méthyl sulfate) 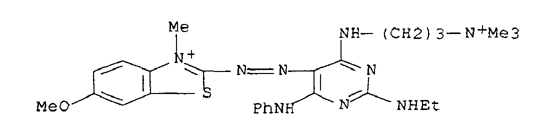
2-[[2-(éthylamino)-4-(phénylamino)-6-[[3- (triméthylammonio) propyl]amino]-5-pyrimidinyl]azo]-6-méthoxy-3-méthyl-benzothiazolium 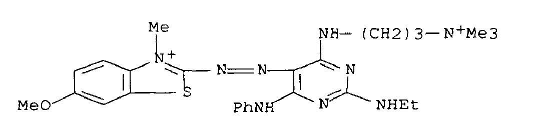
sulfate de 2-[[4-[éthyl[2-(triméthylammonio)éthyl]amino] phényl]azo]-6-méthoxy-3-méthyl- benzothiazolium 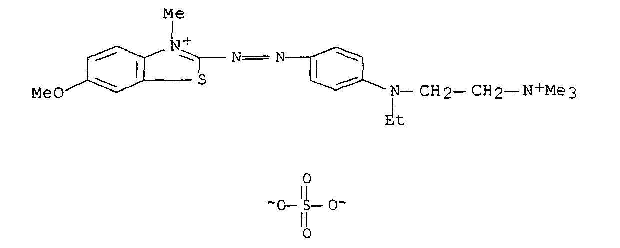
diacétate de 2-[[4-[éthyl[2-(triméthylammonio)éthyl]amino] phényl]azo]-6-méthoxy-3-méthyl- benzothiazolium 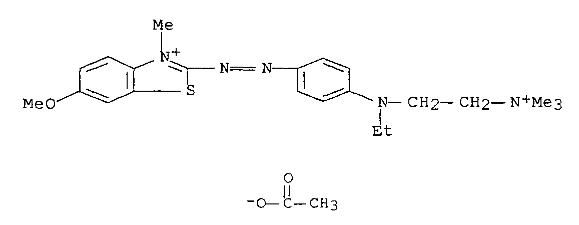
bis(méthyl sulfate) de 2-[[4-[éthyl[2-(triméthylammonio)éthyl] amino]phényl]azo]-6-méthoxy-3-méthyl- benzothiazolium 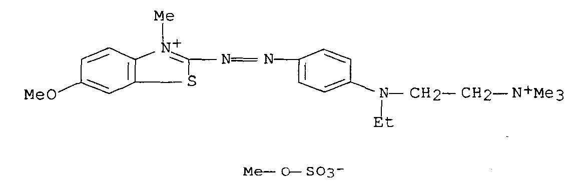
2-[[4-[éthyl[2-(triméthylammonio)éthyl]amino]phényl]azo]-6-méthoxy-3-méthyl-benzothiazolium 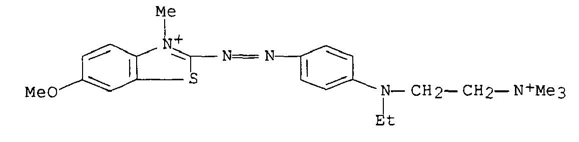
bis[tétrafluoroborate(1-)] de 6-éthoxy-2-[[4-[éthyl[2-hydroxy-3-(triméthylammonio)propyl]amino]-2-méthylphenyl]azo]-3-méthyl-benzothiazolium 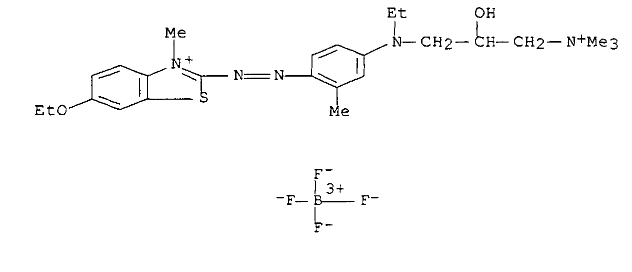
6-éthoxy-2-[[4-[éthyl[2-hydroxy-3- (triméthylammonio)propyl] amino]-2-méthylphényl]azo]-3-méthyl- benzothiazolium 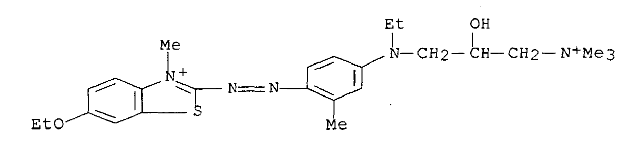
dichlorure de 3-[ 4-(6-méthoxy-3-méthyl-benzothiazol-3-ium) phényl]-3-aza-6-azonia-spiro[5.5]undécane 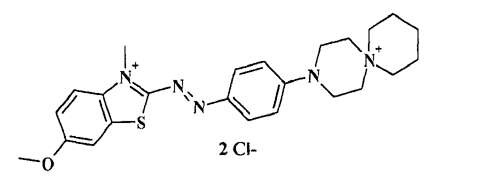
dichlorure de diéthyl-(2-{éthyl-[4-(3-méthyl-benzothiazol-2-ylazo)-phényl]-amino}-éthyl)-(2-phénoxy-éthyl)-ammonium 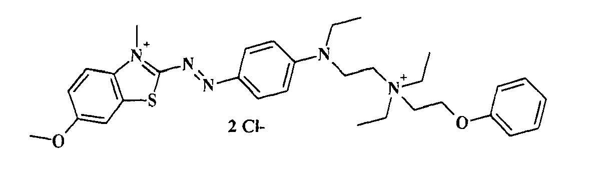
tétrafluoroborate de 1-(2-{éthyl-[4-(6-méthoxy-3-méthyl-benzothiazolium-2-ylazo)-phényl]-amino}-éthyl)-pyridinium 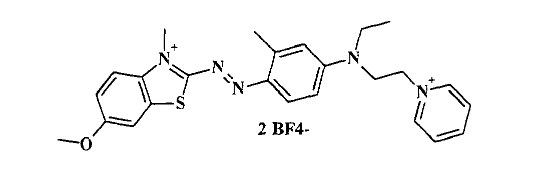
chlorozincate de (2-{éthyl-[4-(1,2,3-triméthyl-1H-benzoimidazolium-5-ylazo)-phényl]-amino}-éthyl)-triméthylammonium 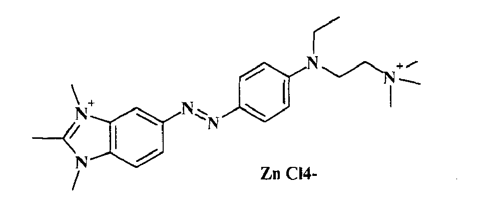
chlorozincate de diéthyl-méthyl-{2-[3-méthyl-4-(1,2,3-triméthyl-1H-benzoimidazolium-5-ylazo)-phényl-amino]-éthyl}-ammonium 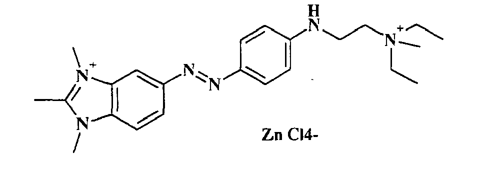
chlorozincate de (2-{propyl-[4-(1,2,3-triméthyl-1H-benzo-imidazolium-5-ylazo)-phényl]-amino}-éthyl)-triméthylammonium 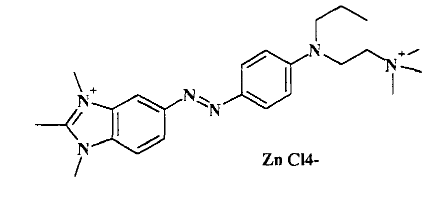
trifluoroborate de (2-{[4-(6-chioro-1,3-diméthyl-3H-benzoimidazolium-4-ylazo)-phényl]-éthyl-amino}-éthyl)-1-méthyl-pyridinium 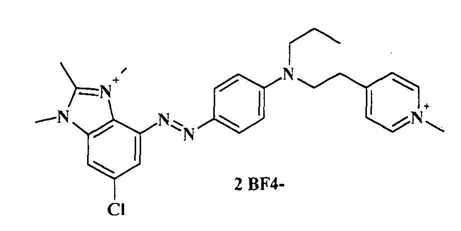
méthosulfate de (2-{4-[5-(3-éthyl-benzothiazolium-2-yl)-6-hydroxy-1-méthyl-2-oxo-1,2-dihydro-pyridin-3-ylazo]-phéayl}-2-oxo-éthyl)-triméthyl-ammonium

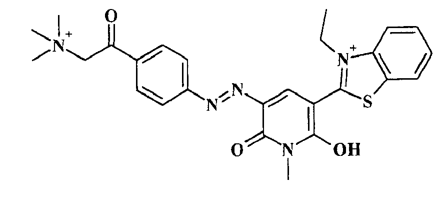

La composition selon la présente demande peut comprendre de 0,001 à 20%, de préférence de 0,05 à 10%, et de manière encore préférée de 0,1 à 5 % en poids de colorant(s) direct(s) de formule (I) par rapport au poids total de la composition.

Les colorants de l'invention peuvent être préparés selon des réactions chimiques connues en soi à partir de chromophores porteurs d'une charge cationique capables de réagir avec le groupement hydrocarboné azoté choisi. Les colorants peuvent notamment être préparés par le procédé décrit dans le document US 3,649,162.

La composition tinctoriale conforme à l'invention peut en outre contenir un ou plusieurs colorants directs additionnels autres que les colorants directs de formule (I) décrits ci-dessus, pouvant être choisis parmi les colorants nitrés de la série benzénique, neutres, acides ou cationiques, les colorants directs azoïques neutres acides ou cationiques, les colorants directs quinoniques et en particulier anthraquinoniques neutres, acides ou cationiques, les colorants directs aziniques, les colorants directs triarylméthaniques, les colorants directs indoaminiques et les colorants directs naturels.

A titre d'exemples de colorants directs additionnels, on peut citer les colorants azoïques cationiques décrits dans les demandes de brevets WO 95/15144, WO-95/01772 et EP-714954.

Parmi ces composés, on peut tout particulièrement citer les colorants suivants :
- chlorure de 1,3-diméthyl-2-[[4-(diméthylamino)phényl]azo]-1H-Imidazolium,
- chlorure de 1,3-diméthyl-2-[(4-aminophényl)azo]-1H-Imidazolium,
- méthylsulfate de 1-méthyl-4-[(méthylphénylhydrazono)méthyl]-pyridinium.

On peut également citer parmi les colorants directs azoïques additionnels les colorants suivants, décrits dans le COLOUR INDEX INTERNATIONAL 3e édition :
- Disperse Red 17
- Acid Yellow 9
- Acid Black 1
- Basic Red 22
- Basic Red 76
- Basic Yellow 57
- Basic Brown 16
- Acid Yellow 36
- Acid Orange 7
- Acid Red 33
- Acid Red 35
- Basic Brown 17
- Acid Yellow 23
- Acid Orange 24
- Disperse Black 9.

Le ou les colorants directs additionnels représentent de préférence de 0,001 à 20% en poids environ du poids total de la composition prête à l'emploi et encore plus préférentiellement de 0,005 à 10% en poids environ.

A titre d'exemple, les bases d'oxydation présentes dans les composition selon l'invention sont choisies parmi les phénylènediamines, les bis-phénylalkylènediamines, les para-aminophénols, les ortho-aminophénols, les bases hétérocycliques autres que les paraphénylènediamines hétérocycliques de formule (I) et leurs sels d'addition.

Parmi les paraphénylènediamines, on peut citer à titre d'exemple, la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylène-diamine, la 4-aminophénylpyrrolidine, la 2-thiényl paraphénylènediamine, le 2-β hydroxyéthylamino 5-amino toluène, la 3-hydroxy 1-(4'-aminophényl)pyrrolidine et leurs sels d'addition avec un acide.

Parmi les paraphénylènediamines citées ci-dessus, la paraphénylènediamine, la paratoluylènediamine, la 2-isopropyl paraphénylènediamine, la 2-β-hydroxyéthyl paraphénylènediamine, la 2-β-hydroxyéthyloxy paraphénylène-diamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, la 2-β-acétylaminoéthyloxy paraphénylènediamine, et leurs sels d'addition avec un acide sont particulièrement préférées.

Parmi les bis-phénylalkylènediamines, on peut citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diamino phénoxy)-3,6-dioxaoctane, et leurs sels d'addition.

Parmi les para-aminophénols, on peut citer à titre d'exemple, le para-aminophénol, le 4-amino 3-méthyl phénol, le 4-amino 3-fluoro phénol, le 4-amino-3-chlorophénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les ortho-aminophénols, on peut citer à titre d'exemple, le 2-amino phénol, le 2-amino 5-méthyl phénol, le 2-amino 6-méthyl phénol, le 5-acétamido 2-amino phénol, et leurs sels d'addition.

Parmi les bases hétérocycliques, on peut citer à titre d'exemple, les dérivés pyridiniques, les dérivés pyrimidiniques et les dérivés pyrazoliques.

Parmi les dérivés pyridiniques, on peut citer les composés décrits par exemple dans les brevets GB 1 026 978 et GB 1 153 196, comme la 2,5-diamino pyridine, la 2-(4-méthoxyphényl)amino 3-amino pyridine, la 3,4-diamino pyridine, et leurs sels d'addition.

D'autres bases d'oxydation pyridiniques utiles dans la présente invention sont les bases d'oxydation 3-amino pyrazolo-[1,5-a]-pyridines ou leurs sels d'addition décrits par exemple dans la demande de brevet FR 2801308. A titre d'exemple, on peut citer la pyrazolo[1,5-a]pyridin-3-ylamine ; la 2-acétylamino pyrazolo-[1,5-a] pyridin-3-ylamine ; la 2-morpholin-4-yl-pyrazolo[1,5-a]pyridin-3-ylamine ; l'acide 3-amino-pyrazolo[1,5-a]pyridin-2-carboxylique ; la 2-méthoxy-pyrazolo[1,5-a]pyridine-3-ylamino ; le (3-amino-pyrazolo[1,5-a]pyridine-7-yl)-méthanol ; le 2-(3-amino-pyrazolo[1,5-a]pyridine-5-yl)-éthanol ; le 2-(3-amino-pyrazolo[1,5-a]pyridine-7-yl)-éthanol ; le (3-amino-pyrazolo[1,5-a]pyridine-2-yl)-méthanol ; la 3,6-diamino-pyrazolo[1,5-a]pyridine ; la 3,4-diamino-pyrazolo[1,5-a]pyridine ; la pyrazolo[1,5-a]pyridine-3,7-diamine ; la 7-morpholin-4-yl-pyrazolo[1,5-a]pyridin-3-ylamine ; la pyrazolo[1,5-a]pyridine-3,5-diamine ; la 5-morpholin-4-yl-pyrazolo[1,5-a]pyridin-3-ylamine ; le 2-[(3-amino-pyrazolo[1,5-a]pyridin-5-yl)-(2-hydroxyéthyl)-amino]-éthanol ; le 2-[(3-amino-pyrazolo[1,5-a]pyridin-7-yl)-(2-hydroxyéthyl)-amino]-éthanol ; la 3-amino-pyrazolo[1,5-a]pyridine-5-ol ; 3-amino-pyrazolo[1,5-a]pyridine-4-ol ; la 3-amino-pyrazolo[1,5-a]pyridine-6-ol ; la 3-amino-pyrazolo[1,5-a]pyridine-7-ol ;
ainsi que leurs sels d'addition.

Parmi les dérivés pyrimidiniques, on peut citer les composés décrits par exemple dans les brevets DE 2359399 ; JP 88-169571 ; JP 05-63124 ; EP 0770375 ou demande de brevet WO 96/15765 comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine et leurs sels d'addition et leurs formes tautomères, lorsqu'il existe un équilibre tautomérique.

Parmi les dérivés pyrazoliques, on peut citer les composés décrits dans les brevets DE 3843892, DE 4133957 et demandes de brevet WO 94/08969, WO 94/08970, FR-A-2 733 749 et DE 195 43 988 comme le 4,5-diamino 1-méthyl pyrazole, le 4,5-diamino 1-(β-hydroxyéthyl) pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition.

La ou les bases d'oxydation présentes dans la composition de l'invention sont en général présentes en quantité comprise allant de 0,001 à 20 % en poids environ du poids total de la composition tinctoriale, de préférence allant de 0,005 à 6 %.

La composition de la présente invention peut en outre comprendre un ou plusieurs précurseurs de colorant d'oxydation choisis parmi les coupleurs.

Les coupleurs peuvent être choisis parmi les coupleurs conventionnellement utilisés pour la teinture de fibres kératiniques. Parmi ces coupleurs, on peut notamment citer les métaphénylènediamines, les métadiphénols, les coupleurs naphtaléniques, les coupleurs hétérocycliques ainsi que leur sels d'addition.

A titre d'exemple, on peut citer le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-β-hydroxyéthylamino-3,4-méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6- hydroxy benzomorpholine la 3,5-diamino-2,6-diméthoxypyridine, le 1-N-(β-hydroxyéthyl)amino-3,4-méthylène dioxybenzène, le 2,6-bis-(β-hydroxyéthylamino)toluène et leurs sels d'addition.

Dans la composition de la présente invention, le ou les coupleurs sont généralement présents en une quantité allant de 0,001 à 20 % en poids du poids total de la composition tinctoriale, de préférence allant de 0,005 à 6 %.

D'une manière générale, les sels d'addition des bases d'oxydation et des coupleurs utilisables dans le cadre de l'invention sont notamment choisis parmi les sels d'addition avec un acide tels que les chlorhydrates, les bromhydrates, les sulfates, les citrates, les succinates, les tartrates, les lactates, les tosylates, les benzènesulfonates, les phosphates et les acétates et les sels d'addition avec une base telles que la soude, la potasse, l'ammoniaque, les amines ou les alcanolamines.

Le milieu approprié pour la teinture appelé aussi support de teinture est un milieu cosmétique généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

Les solvants sont, de préférence, présents dans des proportions de préférence comprises entre 1 et 40 % en poids environ par rapport au poids total de la composition tinctoriale, et encore plus préférentiellement entre 5 et 30 % en poids environ.

La composition tinctoriale conforme à l'invention peut également renfermer divers adjuvants utilisés classiquement dans les compositions pour la teinture des cheveux, tels que des agents tensioactifs anioniques, cationiques, non-ioniques, amphotères, zwitterioniques ou leurs mélanges, des polymères anioniques, cationiques, non-ioniques, amphotères, zwitterioniques ou leurs mélanges, des agents épaississants minéraux ou organiques, et en particulier les épaississants associatifs polymères anioniques, cationiques, non ioniques et amphotères, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Les adjuvants ci dessus sont en général présents en quantité comprise pour chacun d'eux entre 0,01 et 20 % en poids par rapport au poids de la composition.

Bien entendu, l'homme de l'art veillera à choisir ce ou ces éventuels composés complémentaires de manière telle que les propriétés avantageuses attachées intrinsèquement à la composition de teinture d'oxydation conforme à l'invention ne soient pas, ou substantiellement pas, altérées par la ou les adjonctions envisagées.

Le pH de la composition tinctoriale conforme à l'invention est généralement compris entre 3 et 12 environ, et de préférence entre 5 et 11 environ. Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (II) suivante : 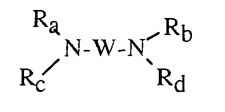 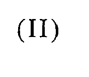 dans laquelle W est un reste propylène éventuellement substitué par un groupement hydroxyle ou un radical alkyle en C₁-C₄ ; Ra, Rb, Rc et Rd, identiques ou différents, représentent un atome d'hydrogène, un radical alkyle en C₁-C₄ ou hydroxyalkyle en C₁-C₄.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

La mise en oeuvre du procédé selon la présente demande comprend l'application d'une composition tinctoriale selon la présente demande sur les fibres kératiniques, puis une étape consistant à laisser poser pendant une période suffisante pour permettre la coloration des cheveux, cette période est généralement comprise entre 5 minutes et 1 heure et de préférence entre 15 minutes et 1 heure.

Le procédé de teinture des fibres kératiniques peut comprendre une étape dans laquelle on utilise un agent oxydant à pH acide, neutre ou alcalin. L'agent oxydant peut être ajouté à la composition de l'invention juste au moment de l'emploi ou il peut être mis en oeuvre à partir d'une composition oxydante le contenant, appliqué simultanément ou séquentiellement à la composition de l'invention.

Ce procédé peut notamment être mis en oeuvre lorsque la composition selon l'invention comprend au moins un précurseur de coloration d'oxydation.

Selon un mode de réalisation particulier, la composition selon la présente invention comprenant au moins un précurseur de colorant d'oxydation est mélangée, de préférence au moment de l'emploi, à une composition contenant, dans un milieu approprié pour la teinture, au moins un agent oxydant, cet agent oxydant étant présent en une quantité suffisante pour développer une coloration. Le mélange obtenu est ensuite appliqué sur les fibres kératiniques. Après un temps de pause de 5 minutes à 1 heure environ, de préférence 15 minutes à 1 heure environ, les fibres kératiniques sont rincées, lavées au shampooing, rincées à nouveau puis séchées.

Les agents oxydants classiquement utilisés pour la teinture des fibres kératiniques sont par exemple le peroxyde d'hydrogène, le peroxyde d'urée, les bromates de métaux alcalins, les persels tels que les perborates et persulfates, les peracides et les enzymes oxydases parmi lesquelles on peut citer les peroxydases, les oxydo-réductases à 2 électrons telles que les uricases et les oxygénases à 4 électrons comme les laccases. Le peroxyde d'hydrogène est particulièrement préféré.

La composition oxydante peut également renfermer divers adjuvants utilisés classiquement dans les compositions pour la teinture des cheveux et tels que définis précédemment.

Dans le mode de réalisation particulier de l'invention où on mélange la composition tinctoriale avec une composition oxydante, le pH de la composition oxydante renfermant l'agent oxydant est tel qu'après mélange avec la composition tinctoriale, le pH de la composition résultante appliquée sur les fibres kératiniques varie de préférence entre 3 et 12 environ, et de préférence entre 5 et 11 environ et de manière encore plus préférée entre 6 et 8,5. Il peut être ajusté à la valeur désirée au moyen d'agents régulateurs de pH, acidifiants ou alcalinisants habituellement utilisés en teinture des fibres kératiniques et tels que définis précédemment.

La composition appliquée sur les fibres kératiniques peut se présenter sous des formes diverses, telles que sous forme de liquides, de crèmes, de gels ou sous toute autre forme appropriée pour réaliser une teinture des fibres kératiniques, et notamment des cheveux humains. En particulier, elle peut être conditionnée sous pression en flacon aérosol en présence d'un agent propulseur et former une mousse.

La présente demande a également pour objet un dispositif à plusieurs compartiments ou "kit" de teinture dans lequel un premier compartiment renferme la composition tinctoriale définie ci-dessus et un deuxième compartiment renferme une composition oxydante. Ce dispositif peut être équipé d'un moyen permettant de délivrer sur les cheveux le mélange souhaité, tel que les dispositifs décrits dans le brevet FR-2 586 913 au nom de la demanderesse.

A partir de ce dispositif, il est possible de teindre les fibres kératiniques à partir d'un procédé qui comprend le mélange d'une composition tinctoriale conforme à l'invention avec un agent oxydant tel que défini précédemment, et l'application du mélange obtenu sur les fibres kératiniques pendant un temps suffisant pour développer la coloration désirée.

Les exemples qui suivent sont destinés à illustrer l'invention, sans toutefois présenter un caractère limitatif.

### Exemple 1

### Composition tinctoriale pour teindre les cheveux en bleu

| | |
|---|---|
| Colorant (1) | 0,15g |
| Ethanol | 10g |
| Alkyl polyglucoside | 5g de matière active |
| 2-amino,2-méthylpropanol | qsp pH 8,0 |
| Eau qsp | 100g |

| | |
|---|---|
| (1) 2-[[4-[éthyl[2-(triméthylammonio)éthyl]amino]phényl]azo]-4- (méthoxycarbonyl)-5-(2-méthoxy-2-oxoéthyl)-3-méthyl-thiazolium | |

### Exemple 2

### Composition tinctoriale pour teindre les cheveux en rouge

| | |
|---|---|
| Colorant (1) | 0,15g |
| Ethanol | 10g |
| Alkyl polyglucoside | 5g de matière active |
| 2-amino,2-méthylpropanol | qsp pH 8,0 |
| Eau qsp | 100g |

| | |
|---|---|
| (1) diiodure de 2-[[4-[[2-(diéthylméthylammonio)éthyl]éthylamino]-2-méthylphenyl]azo]-1,3-diméthyl-1H-imidazolium | |

### Exemple 3

### Composition tinctoriale pour teindre les cheveux en bleu

| | |
|---|---|
| Colorant (1) | 0,15g |
| Ethanol | 10g |
| Alkyl polyglucoside | 5g de matière active |
| 2-amino,2-méthylpropanol | qsp pH 8,0 |
| Eau qsp | 100g |

| | |
|---|---|
| (1) sulfate de 2-[[4-[éthyl[2-(triméthylammonio)éthyl]amino]phényl]azo]-6-méthoxy-3-méthyl-benzothiazolium | |

## Revendications

1. Utilisation à titre de colorant direct dans des compositions tinctoriales pour fibres kératiniques, en particulier les fibres kératiniques humaines telles que les cheveux, ou pour la fabrication de telles compositions, d'un composé de formule (I) : 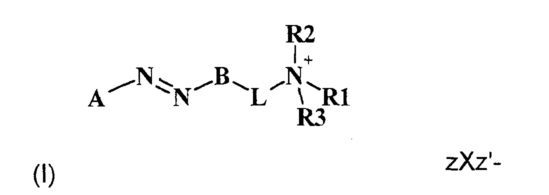 dans laquelle :
- X^{z'}- désigne un anion organique ou minéral ;
- z et z' sont tels que la charge globale de la molécule soit nulle ;
- A désigne un cycle hétérocyclique porteur d'une charge positive ayant la signification thiazolium substitué ou non, les substituants du thiazolium contenant au total au plus une fonction alcoxycarbonyle ; imidazolium substitué ou non ; benzimidazolium substitué ou non ; benzothiazolium pyridino substitué ou non ; naphthothiazolium substitué ou non et benzothiazolium substitué ;
- B désigne un groupement arylène en C6-C30 éventuellement substitué ou un groupe hétérocyclique de 5 à 10 chaînons éventuellement substitué ;
- L désigne une chaîne alkylène linéaire, ramifiée ou cyclique, substituée ou non et comportant de 1 à 30 atomes de carbone, la chaîne pouvant être ou non interrompue ou terminée par un ou plusieurs groupements hétéroatomiques choisi parmi O, S, NH, NR avec R représentant un radical alkyle en C1-C10 linéaire ou ramifié, ou interrompue par un ou plusieurs radicaux cycliques, aromatiques ou non, hétérocycliques ou non, la chaîne elle-même pouvant être porteuse d'une ou plusieurs charges cationiques quaternaires et un ou plusieurs atomes de carbone de la chaîne pouvant être remplacés par un groupement carbonyle ;
- R1, R2, R3, désignent indépendamment l'un de l'autre un radical alkyle en C1-C10 linaire, ramifié ou cyclique éventuellement substitué ; un radical arylalkyle en C1-C10 linaire, ramifié éventuellement substitué ; un radical aryle, éventuellement substitué ; un radical aryloxyalkyle en C1-C10 linaire, ramifié ; l'un au moins des radicaux R1, R2, R3 pouvant former avec l'atome d'azote le(s) portant et éventuellement avec L un hétérocycle saturé ou insaturé ayant de 5 à 10 chaînons éventuellement substitué pouvant contenir un ou plusieurs hétéroatomes supplémentaires.

2. Utilisation selon la revendication 1, **caractérisée en ce que** le composé de formule (I) est choisi parmi :
- le 2-[[4-[éthyl[2-(triméthylammonio)éthyl]amino]phényl]azo]-4- (méthoxycarbonyl)-5-(2-méthoxy-2-oxoéthyl)-3-méthyl-thiazolium,
- le dichlorure de thiazolium, 2-[[4-[[2-[diméthyl(phénylméthyl)ammonio]éthyl]éthylamino]phényl]azo]-3-méthyl-thiazolium,
- le 2-[[4-(diéthylamino)-2-[[(triméthylammonio)acétyl]amino]phényl]azo]-5-(éthoxycarbonyl)-3-méthyl-4-phényl-thiazolium,
- le bis[tétrafluoroborate(1-)] de 2-[[4-(diéthylamino)-2-[[(triméthylammonio)acétyl]oxy]phényl]azo]-5-(éthoxycarbonyl)-3,4-diméthyl-thiazolium,
- le 2-[[4-(diéthylamino)-2-[[(triméthylammonio)acétyl]oxy]phényl]azo]-5-(éthoxycarbonyl)-3,4-diméthyl-thiazolium,
- le dichlorure de 2-[[4-[éthyl[2-[(2-hydroxypropyl)diméthylammonio]éthyl]amino]-2-méthylphényl]azo]-3-méthyl-thiazolium,
- le dichlorure de 2-[[4-[éthyl[2-(triméthylammonio)éthyl]amino]phényl]azo]-3-méthyl-thiazolium,
- le bis[tétrafluoroborate(1-)] de 2-[[4-[éthyl[2-(triméthylammonio)éthyl]amino]phényl]azo]-3-méthyl-4-phényl-thiazolium,
- le 2-[[4-[éthyl[2-(triméthylammonio)éthyl]amino]phényl]azo]-3-méthyl-4-phényl-thiazolium,
- le chlorure de 1-(2-{(4-méthoxy-phényl)-[3-méthyl-4-(thiazol-3-ium-2-ylazo)-phényl]-amino}-ethyl)-4-méthyl-pyridinium,
- le chlorozincate de 1-(2-{phényl-[5-éthoxycarbonyl-3,4-diméthyl-4-(thiazol-3-ium-2-ylazo)-phényl]-amino}-éthyl)-pyridinium,
- le chlorure de 1-(2-{(4-méthoxy-phényl)-[5-éthoxycarbonyl-3,4-diméthyl-4-(thiazol-3-ium-2-ylazo)-phényl]-amino}-éthyl)-4-méthyl-pyridinium,
- le chlorozincate de 4-diméthylamino-1-(2-{[4-(5-éthoxycarbonyl-3,4-diméthyl-4-(thiazol-3-ium-2-ylazo)-phényl]-p-tolyl-amino}-éthyl) pyridinium,
- le chlorure de 1-{2-[[4-(5-acétyl-3,4-diméthyl-4-(thiazol-3-ium-2-ylazo )-phényl]-(4-méthoxy-phényl)-amino]-éthyl-pyridinium,
- le chlorozincate de 1-(3-{[4-(5-éthoxycarbonyl-3,4-diméthyl-4-(thiazol-3-ium-2-ylazo)-phényl]-phényl-amino}-2-hypropyl)-pyridinium,
- le chlorure 1-{3-[[4-(5-éthoxycarbonyl-3,4-diméthyl-4-(thiazol-3-ium-2-ylazo)-phényl]-(4-méthoxy-phényl)-2-hydroxypropyl)-4- méthyl-pyridinium,
- le diiodure de 2-[[4-[éthyl[2-[[(triméthylammonio)acétyl]amino]éthyl]amino]-2-méthylphényl]azo]-1,3-diméthyl-1H-imidazolium,
- le diiodure de 2-[[4-[[3-(diéthylméthylammonio)-2-hydroxypropyl]éthylamino]-2-méthylphényl]azo]-1,3-diméthyl-1H-imidazolium,
- le diiodure de 2-[[4-[[2-(diéthylméthylammonio)éthyl]éthylamino]-2-méthylphenyl]azo]-1,3-diméthyl-1H-imidazolium,
- le diiodure de 2-[[4-[éthyl[2-(triméthylammonio)éthyl]amino]phényl]azo]-1,3-diméthyl-1H-imidazolium,
- le diiodure de 2-[[4-[[2-(diéthylméthylammonio)éthyl]éthylamino]phényl]azo]-1,3-diméthyl-1H-imidazolium,
- le dichlorure de 2-[[4-[éthyl[2-(triméthylammonio)éthyl]amino]phényl]azo]-3-méthyl-naphtho[2,1-d]thiazolium,
- le 2-[[4-anilino-2-(éthylamino)-6-[[3-(triméthylammonio)propyl]amino]-5-pyrimidinyl]azo]-6-méthoxy-3-méthyl-benzothiazolium, bis(méthyl sulfate),
- le 2-[[2-(éthylamino)-4-(phénylamino)-6-[[3-(triméthylammonio)propyl]amino]-5-pyrimidinyl]azo]-6-méthoxy-3-méthyl-benzothiazolium,
- le sulfate de 2-[[4-[éthyl[2-(triméthylammonio)éthyl]amino]phényl]azo]-6-méthoxy-3-méthyl-benzothiazolium,
- le diacétate de 2-[[4-[éthyl[2-(triméthylammonio)éthyl]amino]phényl]azo]-6-méthoxy-3-méthyl-benzothiazolium,
- le bis(méthyl sulfate) de 2-[[4-[éthyl[2-(triméthylammonio)éthyl]amino]phényl]azo]-6-méthoxy-3-méthyl-benzothiazolium,
- le 2-[[4-[éthyl[2-(triméthylammonio)éthyl]amino]phényl]azo]-6-méthoxy-3-méthyl-benzothiazolium,
- le bis[tétrafluoroborate(1-)] de 6-éthoxy-2-[[4-[éthyl[2-hydroxy-3-(triméthylammonio)propyl]amino]-2-méthylphenyl]azo]-3-méthyl- benzothiazolium,
- le 6-éthoxy-2-[[4-[éthyl[2-hydroxy-3-(triméthylammonio)propyl]amino]-2-méthylphényl]azo]-3-méthyl-benzothiazolium,
- le dichlorure de 3-[ 4-(6-méthoxy-3-méthyl-benzothiazol-3-ium) phényl]-3-aza-6-azonia-spiro[5.5]undécane,
- le dichlorure de diéthyl-(2-{éthyl-[4-(3-méthyl-benzothiazol-2-ylazo)-phényl]-amino}-éthyl)-(2-phénoxy-éthyl)-ammonium,
- le tétrafluoroborate de 1-(2-{éthyl-[4-(6-méthoxy-3-méthyl-benzothiazolium-2-ylazo)-phényl]-amino}-éthyl)-pyridinium,
- le chlorozincate de (2-{éthyl-[4-(1,2,3-triméthyl-1H-benzoimidazolium-5-ylazo)-phényl]-amino}-éthyl)-triméthylammonium,
- le chlorozincate de diéthyl-méthyl-{2-[3-méthyl-4-(1,2,3-triméthyl-1H-benzoimidazolium-5-ylazo)-phényl-amino]-éthyl}-ammonium,
- le chlorozincate de (2-{propyl-[4-(1,2,3-triméthyl-1H-benzoimidazolium-5-ylazo)-phényl]-amino}-éthyl)-triméthylammonium,
- le trifluoroborate de (2-{[4-(6-chloro-1,3-diméthyl-3H-benzoimidazoiuml-4-ylazo)-phényl]-éthyl-amino}-éthyl)-1-méthyl-pyridinium,
- le méthosulfate de (2-{4-[5-(3-éthyl-benzothiazolium-2-yl)-6-hydroxy-1-méthyl-2-oxo-1,2-dihydro-pyridin-3-ylazo]-phényl}-2-oxo-éthyl)-triméthyl-ammonium.

3. Composition tinctoriale pour la teinture des fibres kératiniques, en particulier des fibres kératiniques humaines telles que les cheveux comprenant dans un milieu de teinture approprié au moins une base d'oxydation et au moins un composé de formule (I) tel que défini dans la revendication 1.

4. Composition selon la revendication 3, **caractérisée en ce que** le composé de formule (I) est choisi parmi :
- le 2-[[4-[éthyl[2-(triméthylammonio)éthyl]amino]phényl]azo]-4- (méthoxycarbonyl)-5-(2-méthoxy-2-oxoéthyl)-3-méthyl-thiazolium,
- le dichlorure de thiazolium, 2-[[4-[[2-[diméthyl(phénylméthyl)ammonio]éthyl]éthylamino]phényl]azo]-3-méthyl-thiazolium,
- le 2-[[4-(diéthylamino)-2-[[(triméthylammonio)acétyl]amino]phényl]azo]-5-(éthoxycarbonyl)-3-méthyl-4-phényl-thiazolium,
- le bis[tétrafluoroborate(1-)] de 2-[[4-(diéthylamino)-2-[[(triméthylammonio)acétyl]oxy]phényl]azo]-5-(éthoxycarbonyl)-3,4-diméthyl-thiazolium,
- le 2-[[4-(diéthylamino)-2-[[(triméthylammonio)acétyl]oxy]phényl]azo]-5-(éthoxycarbonyl)-3,4-diméthyl-thiazolium,
- le dichlorure de 2-[[4-[éthyl[2-[(2-hydroxypropyl)diméthylammonio]éthyl]amino]-2-méthylphényl]azo]-3-méthyl-thiazolium,
- le dichlorure de 2-[[4-[éthyl[2-(triméthylammonio)éthyl]amino]phényl]azo]-3-méthyl-thiazolium,
- le bis[tétrafluoroborate(1-)] de 2-[[4-[éthyl[2-(triméthylammonio)éthyl]amino]phényl]azo]-3-méthyl-4-phényl-thiazolium,
- le 2-[[4-[éthyl[2-(triméthylammonio)éthyl]amino]phényl]azo]-3-méthyl-4-phényl-thiazolium,
- le chlorure de 1-(2-{(4-méthoxy-phényl)-[3-méthyl-4-(thiazol-3-ium-2-ylazo)-phényl]-amino}-ethyl)-4-méthyl-pyridinium,
- le chlorozincate de 1-(2-{phényl-[5-éthoxycarbonyl-3,4-diméthyl-4-(thiazol-3-ium-2-ylazo)-phényl]-amino}-éthyl)-pyridinium,
- le chlorure de 1-(2-{(4-méthoxy-phényl)-[5-éthoxycarbonyl-3,4-diméthyl-4-(thiazol-3-ium-2-ylazo)-phényl]-amino}-éthyl)-4-méthyl-pyridinium,
- le chlorozincate de 4-diméthylamino-1-(2-{[4-(5-éthoxycarbonyl-3,4-diméthyl-4-(thiazol-3-ium-2-ylazo)-phényl]-p-tolyl-amino}-éthyl) pyridinium,
- le chlorure de 1-{2-[[4-(5-acétyl-3,4-diméthyl-4-(thiazol-3-ium-2-ylazo )-phényl]-(4-méthoxy-phényl)-amino]-éthyl-pyridinium,
- le chlorozincate de 1-(3-{[4-(5-éthoxycarbonyl-3,4-diméthyl-4-(thiazol-3-ium-2-ylazo)-phényl]-phényl-amino}-2-hypropyl)-pyridinium,
- le chlorure 1-{3-[[4-(5-éthoxycarbonyl-3,4-diméthyl-4-(thiazol-3-ium-2-ylazo)-phényl]-(4-méthoxy-phényl)-2-hydroxypropyl)-4- méthyl-pyridinium,
- le diiodure de 2-[[4-[éthyl[2-[[(triméthylammonio)acétyl]amino]éthyl]amino]-2-méthylphényl]azo]-1,3-diméthyl-1H-imidazolium,
- le diiodure de 2-[[4-[[3-(diéthylméthylammonio)-2-hydroxypropyl]éthylamino]-2-méthylphényl]azo]-1,3-diméthyl-1H-imidazolium,
- le diiodure de 2-[[4-[[2-(diéthylméthylammonio)éthyl]éthylamino]-2-méthylphenyl]azo]-1,3-diméthyl-1H-imidazolium,
- le diiodure de 2-[[4-[éthyl[2-(triméthylammonio)éthyl]amino]phényl]azo]-1,3-diméthyl-1H-imidazolium,
- le diiodure de 2-[[4-[[2-(diéthylméthylammonio)éthyl]éthylamino]phényl]azo]-1,3-diméthyl-1H-imidazolium,
- le dichlorure de 2-[[4-[éthyl[2-(triméthylammonio)éthyl]amino]phényl]azo]-3-méthyl-naphtho[2,1-d]thiazolium,
- le 2-[[4-anilino-2-(éthylamino)-6-[[3-(triméthylammonio)propyl]amino]-5-pyrimidinyl]azo]-6-méthoxy-3-méthyl-benzothiazolium, bis(méthyl sulfate),
- le 2-[[2-(éthylamino)-4-(phénylamino)-6-[[3-(triméthylammonio)propyl]amino]-5-pyrimidinyl]azo]-6-méthoxy-3-méthyl-benzothiazolium,
- le sulfate de 2-[[4-[éthyl[2-(triméthylammonio)éthyl]amino]phényl]azo]-6-méthoxy-3-méthyl-benzothiazolium,
- le diacétate de 2-[[4-[éthyl[2-(triméthylammonio)éthyl]amino]phényl]azo]-6-méthoxy-3-méthyl-benzothiazolium,
- le bis(méthyl sulfate) de 2-[[4-[éthyl[2-(triméthylammonio)éthyl]amino]phényl]azo]-6-méthoxy-3-méthyl-benzothiazolium,
- le 2-[[4-[éthyl[2-(triméthylammonio)éthyl]amino]phényl]azo]-6-méthoxy-3-méthyl-benzothiazolium,
- le bis[tétrafluoroborate(1-)] de 6-éthoxy-2-[[4-[éthyl[2-hydroxy-3-(triméthylammonio)propyl]amino]-2-méthylphenyl]azo]-3-méthyl- benzothiazolium,
- le 6-éthoxy-2-[[4-[éthyl[2-hydroxy-3-(triméthylammonio)propyl]amino]-2-méthylphényl]azo]-3-méthyl-benzothiazolium,
- le dichlorure de 3-[ 4-(6-méthoxy-3-méthyl-benzothiazol-3-ium) phényl]-3-aza-6-azonia-spiro[5.5]undécane,
- le dichlorure de diéthyl-(2-{éthyl-[4-(3-méthyl-benzothiazol-2-ylazo)-phényl]-amino}-éthyl)-(2-phénoxy-éthyl)-ammonium,
- le tétrafluoroborate de 1-(2-{éthyl-[4-(6-méthoxy-3-méthyl-benzothiazolium-2-ylazo)-phényl]-amino}-éthyl)-pyridinium,
- le chlorozincate de (2-{éthyl-[4-(1,2,3-triméthyl-1H-benzoimidazolium-5-ylazo)-phényl]-amino}-éthyl)-triméthylammonium,
- le chlorozincate de diéthyl-méthyl-{2-[3-méthyl-4-(1,2,3-triméthyl-1H-benzoimidazolium-5-ylazo)-phényl-amino]-éthyl}-ammonium,
- le chlorozincate de (2-{propyl-[4-(1,2,3-triméthyl-1H-benzoimidazolium-5-ylazo)-phényl]-amino}-éthyl)-triméthylammonium,
- le trifluoroborate de (2-{[4-(6-chloro-1,3-diméthyl-3H-benzoimidazoiuml-4-ylazo)-phényl]-éthyl-amino}-éthyl)-1-méthyl-pyridinium,
- le méthosulfate de (2-{4-[5-(3-éthyl-benzothiazolium-2-yl)-6-hydroxy-1-méthyl-2-oxo-1,2-dihydro-pyridin-3-ylazo]-phényl}-2-oxo-éthyl)-triméthyl-ammonium.

5. Composition selon la revendication 3 ou 4, **caractérisée en ce qu'**elle comprend de 0,001 à 20%, de préférence de 0,05 à 10%, et de manière encore préférée de 0,1 à 5% en poids de colorant(s) direct(s) de formule (I) par rapport au poids total de la composition.

6. Composition tinctoriale selon l'une des revendications 3 à 5, **caractérisée en ce que** la base d'oxydation est choisie parmi les paraphénylènediamines, les bis-phénylalkylènediamines, les paraaminophénols, les orthoaminophénols, les bases hétérocycliques et leurs sels d'addition.

7. Composition tinctoriale selon l'une des revendications 3 à 6, **caractérisée en ce que** la ou les bases d'oxydation sont présentes en une quantité comprise entre 0,001 à 20 % en poids et de préférence entre 0,005 et 6 % en poids par rapport au poids total de la composition.

8. Composition selon l'une des revendications 3 à 7, **caractérisée en ce qu'**elle comprend au moins un colorant direct additionnel.

9. Composition selon l'une des revendications 3 à 8, **caractérisée en ce qu'**elle contient au moins un précurseur de colorant d'oxydation choisi parmi les coupleurs.

10. Composition selon la revendication 9, **caractérisée en ce que** le coupleur est choisi parmi les métaphénylènediamines, les métaaminophénols, les métadiphénols, les coupleurs naphtaléniques, les coupleurs hétérocycliques et leurs sels d'addition.

11. Composition selon la revendication 9, **caractérisée en ce que** le coupleur est choisi parmi le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-β-hydroxyéthylamino-3,4-méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6- hydroxy benzomorpholine la 3,5-diamino-2,6-diméthoxypyridine, le 1-N-(β-hydroxyéthyl)amino-3,4-méthylène dioxybenzène, le 2,6-bis-(β-hydroxyéthylamino)toluène et leurs sels d'addition.

12. Composition selon l'une des revendications 9 à 11, **caractérisée en ce que** le ou les coupleurs sont présents en une quantité comprise entre 0,001 et 20 % de préférence entre 0,005 et 6 % en poids par rapport au poids total de la composition.

13. Composition selon l'une des revendications 3 à 12, **caractérisée en ce qu'**elle comprend au moins un solvant.

14. Composition selon la revendication 13, **caractérisée en ce que** le solvant est choisi parmi l'éthanol, le propylène glycol, le glycérol, et les monoéthers de polyols.

15. Composition selon l'une des revendications 3 à 14, **caractérisée en ce qu'**elle comprend au moins un adjuvant choisi parmi les tensioactifs anioniques, cationiques, non ioniques, amphotères, zwitterioniques ou leurs mélanges, les polymères anioniques, cationiques, non ioniques, amphotères, zwitterioniques ou leurs mélanges, les agents épaississants minéraux ou organiques, les agents antioxydants, les agents de pénétration, les agents séquestrants, les parfums, les tampons, les agents dispersants, les agents conditionneurs, les agents filmogènes, les céramides, les agents conservateurs, les agents opacifiants.

16. Composition selon l'une des revendications 3 à 15, **caractérisée en ce qu'**elle comprend au moins un agent oxydant choisi parmi le peroxyde d'hydrogène, le peroxyde d'urée, les bromates de métaux alcalins, les persels, les peracides et les enzymes oxydases.

17. Composition selon la revendication 16, **caractérisée en ce que** l'agent oxydant est le peroxyde d'hydrogène.

18. Procédé de teinture des fibres kératiniques, **caractérisé en ce qu'**il comprend l'application d'une composition selon l'une des revendications 3 à 17, sur les fibres kératiniques, puis une étape consistant à laisser poser pendant une période comprise entre 5 minutes et une heure, de préférence entre 15 minutes et 1 heure.

19. Utilisation d'une composition selon l'une des revendications 3 à 17 pour la teintures des fibres kératiniques, en particulier des fibres kératiniques humaines telles que les cheveux.

20. Utilisation d'une composition selon l'une des revendications 3 à 17 sur les des fibres kératiniques, en particulier les fibres kératiniques humaines telles que les cheveux, pour obtenir des teintures présentant une bonne résistance aux agents extérieurs et aux shampoings.
